# EUROPÄISCHE PATENTANMELDUNG

(11) **EP 0 962 214 A1**
(43) Veröffentlichungstag der Anmeldung: **08.12.1999**
(21) Anmeldenummer: 99110323.5
(22) Anmeldetag: 27.05.1999
(51) Int. Cl.: A61K 6/083

(54) **Dentales Restaurationsmaterial mit ästhetisch wirkenden Beimengungen**

(30) Priorität: 29.05.1998 DE 29809750 U
(71) Anmelder: VOCO GmbH, 27472 Cuxhaven (DE)
(72) Erfinder: Maletz, Reinhard, Dr., 27472 Cuxhaven (DE); Plaumann, Manfred Thomas, 27476 Cuxhaven (DE)

(57) **Zusammenfassung**

In zunehmenden Maße wird der Zahnarzt mit dem Problem individueller Wünsche und besonderen Gegebenheiten der zu behandelnden Patienten konfrontiert, die eine von der Allgemeinheit abweichende, differenzierte, farbige Restauration wünschen. Dieser Wunsch läßt sich aus kosmetischen, modischen oder individuellen Gesichtspunkten heraus begründen. In das erfindungsgemäße dentale Restaurationsmaterial sind neben weiteren üblichen Bestandteilen anorganische und/oder organische Farbmittel zur Erzielung einer nicht zahnfarbenen Farbwirkung eingearbeitet. Die Einarbeitung von Farbmitteln ermöglicht beispielsweise eine farbige, nicht zahnfarbene, Anpassung des Restaurationsmaterials an die Zahnumgebung wie das Zahnfleisch.

## Beschreibung

Die Erfindung betrifft dentale Restaurationsmaterialien mit ästhetisch wirkenden Beimengungen zum Ersetzen oder Restaurieren von Zähnen. In das Restaurationsmaterial sind neben weiteren üblichen Bestandteilen anorganische und/oder organische Farbmittel zur Erzielung einer nicht zahnfarbenen Farbwirkung eingearbeitet.

Ein wichtiger Aspekt der zahnärztlichen Behandlung ist aus der Sicht des Patienten häufig die Farbe und das Aussehen der Zähne. In unserem täglichen Leben spielen Farben eine bedeutende Rolle. Die Ästhetische Zahnmedizin kann das Erscheinungsbild eines Menschen entscheidend verändern, Bislang wird bei der dentalen Restaurationstechnik aus ästhetischen Gründen versucht, eine Anpassung der Restaurationsfarbe an die Farbe der natürlichen Zähne zu erzielen, wie es beispielsweise in der EP-PS 424391 beschrieben wird. Dies wird durch einen Zusatz sogenannter Opaker bzw. Grundiermassen erreicht. Damit die Opaker in einem weiten Bereich eine farbige Anpassung an natürliches Zahnmaterial gestatten, enthalten die Opaker üblicherweise weißes Pigment auf TiO₂- oder ZrO₂-Basis. Dentale Opaker sind aus dem Stand der Technik bekannt, wie in DE-OS 19635667 angegeben. In der DE-OS 4119483 werden zur Einfärbung der pastenförmigen Grundiermassen gegebenenfalls Farbpigmente zugemischt um eine farbliche Anpassung an die natürliche Zahnfarbe zu erreichen. Bei dentalen Füllungsmaterialien mit bekannten Opakern, die durch Licht gehärtet werden, tritt dabei das Problem auf, daß diese infolge des Gehaltes an abdeckenden Pigmenten wie TiO₂ bzw. ZrO₂ nur in unbefriedigendem Maße bei Bestrahlung mit Licht aushärten. Lichtdurchlässige Systeme werden beispielsweise in der EP-OS 686674 offenbart. Darin sind Farbmittel (Farbstoffe, Pigmente) mit vom Betrachtungswinkel abhängiger Farbigkeit zu einem lichtdurchlässigen Gesamtsystem verarbeitet worden.

Allen bekannten Druckschriften gemäß dem Stand der Technik liegt die Forderung und Aufgabe zugrunde, daß aus ästhetischen und/oder funktionellen Gründen das Restaurationsmaterial eine möglichst große Ähnlichkeit mit den natürlichen Zähnen aufweisen soll oder aber daß es zumindest eventuelle Metallgerüste überdeckt.

Dabei hat der Patient seine eigene Vorstellung von schönen, ästhetischen Zähnen und der richtigen Farbwahl. Der Zahnarzt oder Zahntechniker ist dem Patienten gegenüber verpflichtet sich seinen Wünschen und Vorstellungen offen zu zeigen und diese mit den Mitteln im Rahmen der medizinischen Vertretbarkeit zu erfüllen. In zunehmenden Maße wird der Zahnarzt mit dem Problem individueller Wünsche und besonderen Gegebenheiten der zu behandelnden Patienten konfrontiert, die eine von der Allgemeinheit abweichende, differenzierte Restauration wünschen. Dieser Wunsch läßt sich aus kosmetischen, modischen oder individuellen Gesichtspunkten heraus begründen. Derzeit wird diesem Wunsch beispielsweise durch das Anbringen von Schmuckstücken, wie Diamanten o.ä., auf den Schneidezähnen entsprochen (Quintessenz Zahntechnik 24, 3 225 ff. 1998).

Als weiteres Problem bei der Auswahl des gewünschten Füllungsmaterials tritt der Kostenaspekt mehr und mehr in den Vordergrund. Eine Goldrestauration ist aus zahnmedizinischer Sicht eine der langlebigsten und sichersten Restaurationsmöglichkeiten, so daß seitens des Patienten häufig eine hochwertige Goldrestauration gewünscht wird. Durch die Kostensteigerungen und die zunehmende Selbstkostenbeteiligung bei der zahnmedizinischen Versorgung können aber viele Patienten eine solche hochwertige Restauration sich nicht mehr leisten. Dies birgt das Problem in sich, daß zu präparierende Zähne, die neben einer Goldrestauration liegen, mit einer weißen, zahnfarbenen oder metallischen, z.B. Amalgam, Füllung versehen werden müssen.

Die zunehmende "Überalterung" der Bevölkerung in den industrialisierten Staaten bringt weitere zahnmedizinische Probleme mit sich. Durch Erfolge in der Kariesprävention besitzen viele ältere Menschen zunehmend noch eigene Zähne, dennoch tritt aufgrund vieler verschiedener altersbedingter Veränderungen Zahnfleischschwund auf. Damit häufen sich Probleme mit freiliegenden Zahnhälsen, die einen unästhetischen Anblick bieten. Zudem kann es aufgrund der altersbedingten Veränderungen (Zahnfleischschwund, verringerter Speichelfluß) verstärkt zu kariösen Zahnhals- oder Wurzelläsionen und Dentinempfindlichkeiten kommen. Bislang wird zur Abdeckung von freiliegenden Zahnhälsen eine Zahnfleischepithese angebracht, die neben den hohen Kosten eine unangenehme und aufwendige Verarbeitung und einen ungünstigen Tragekomfort zur Folge hat. Darüber hinaus sind damit keinerlei prophylaktische Maßnahmen in Bezug auf Zahnhals- und/oder Wurzelkaries vorgenommen worden.

Aufgabe der Erfindung ist es deshalb, ein dentales Restaurationsmaterial zur Verfügung zu stellen, das die genannten Nachteile vermeidet und den individuellen Wünschen des Patienten nahekommt. Insbesondere ist es die Aufgabe der Erfindung ein dentales Restaurationsmaterial bereit zu stellen, das neben prophylaktischen und prothetischen Zwecken auch ästhetischen und modischen Gesichtspunkten gerecht wird.

Die Lösung der Aufgabe wird durch ein Restaurationsmaterial nach dem Oberbegriff des Anspruches 1 wiedergegeben. In das erfindungsgemäße Restaurationsmaterial sind anorganische und/oder organische Farbmittel eingearbeitet, so daß das Restaurationsmaterial entsprechend den individuellen Wünschen und Gegebenheiten farbige, nicht zahnfarbene, Farbwirkungen erzielt. Es werden dabei keinerlei Einbußen hinsichtlich der Verarbeitung, der Anwendung und den chemischen, physikalischen und prophylaktischen Eigenschaften des Restaurationsmaterials festgestellt. Insbesondere werden die Festigkeit, Löslichkeit und Verarbeitung durch die Einarbeitung der Farbmittel nicht beeinflußt. Beispielsweise als goldfarbene Variante ist das erfindungsgemäße Füllmaterial neben vorhandenen Goldrestaurationen einzusetzen. Als zahnfleischfarbene Variante ist es insbesondere geeignet, freiliegende Zahnhals- oder Wurzeldentindefekte zu restaurieren.
Besonders ästhetische Farbwirkungen, die gerade in der Kinderzahnheilkunde eine große Rolle spielen, werden dadurch erreicht, daß dem Restaurationsmaterial Farbmittel eingearbeitet sind, so daß ein gelbes, grünes, blaues, rotes oder durch Mischung mehrerer Farbmittel bunt gefärbtes Restaurationsmaterial vorliegt. Ein weiterer, besonders ästhetischer Effekt, der wiederum für Kinder besonders ansprechend wirkt, wird durch die Einarbeitung von Farbmitteln erreicht, so daß das Restaurationsmaterial einen Glimmer- oder glimmerartigen Anteil aufweist.

Die Erfindung umfaßt auch das Verfahren zur Herstellung farbiger, nicht zahnfarbener, dentaler Restaurationsmaterialien, indem in das Restaurationsmaterial neben weiteren üblichen Bestandteilen anorganische und/oder organische Farbmittel zur Erzielung einer nicht zahnfarbenen Farbwirkung eingearbeitet werden.
Des weiteren umfaßt die Erfindung die Verwendung farbiger, nicht zahnfarbener, dentaler Restaurationsmaterialien zum Ersetzen oder Restaurieren von Zähnen, indem in das Restaurationsmaterial neben weiteren üblichen Bestandteilen anorganische und/oder organische Farbmittel zur Erzielung einer nicht zahnfarbenen Farbwirkung eingearbeitet sind.
Es ist selbstverständlich, daß die mit dem erfindungsgemäßen Restaurationsmaterial versorgten Zähne die entsprechenden ästhetischen, farbigen, nicht zahnfarbenen Farbwirkungen aufweisen.

Die Herstellung, Eigenschaften und Vorteile dentaler, zahnfarbener Restaurationsmaterialien sind vom Stand der Technik bekannt, wie beispielsweise nach EP-PS 219058. Bekannt sind dentale Restaurationsmaterialien auf Basis von Composite-Materialien oder Glasionomerzementen. Durch die erfindungsgemäßen, ästhetisch wirkenden Beimengungen von Farbmitteln werden die Herstellung, die Eigenschaften und die Vorteile der jeweiligen Restaurationsmaterialien nicht verändert. Zahnärztliche Restaurationskunststoffe sind beispielsweise synthetische Kunststoffe, die entsprechend dem Zugrunde liegenden Reaktionstyp als Polykondensate, Polyaddukte oder Polymerisate bezeichnet werden. Die Polymerisate der Acryl-, Methacrylsäure und deren Derivate bilden die Grundlage der modernen Composite-Kunststoffe. Allgemein wird der Ausdruck Composite auf die angloamerikanische Bezeichnung "composite resin material" zurückgeführt und bezeichnet Stoffe, die zusätzlich zu den makromolekularen organischen Polymeren weitere Bestandteile, in der Regel anorganische Partikel, als Füllstoffe enthalten. Dentale Harzsysteme auf Acrylat- bzw. Methacrylatbasis sind für die meisten zahnärztlichen Anwendungen das Material der Wahl. Dies begründet sich mit den zweckmäßigen Eigenschaften dieser Stoffklasse. Neben der erforderlichen Festigkeit, Abrasionsresistenz, Dimensionsstabilität, Biokompatibilität und Ästhetik zählen vor allem die anwendungsfreundliche Herstellung und Verarbeitung zu den wesentlichen Materialvorteilen.

Als Farbmittel können übliche, zur Einfärbung von Dentalwerkstoffen geeignete Farbstoffe und/oder Farbpigmente verwendet werden. Insbesondere: Ultramarinblau, Eisenoxid-Pigmente, Titandioxid-Pigmente, farbige Pigmente auf Basis von Co, Al, Cr, Ni, und/oder Zn-oxiden, organische farbige Pigmente sowie Glimmer. Eine Auswahl bekannter Farbmittel findet sich beispielsweise im Merck Katalog 1997 Reagenzien-Chemikalien-Diagnostica. Die Einarbeitung der Farbmittel in die erfindungsgemäßen dentalen Restaurationsmaterialien erfolgt nach dem Stand der Technik, beispielsweise wie in EP-OS 686674 beschrieben. Als im sichtbaren Wellenlängenbereich eingesetzte Farbmittel (Farbstoffe, Pigmente) werden die anorganischen oder organischen Stoffe in Mengen von 0,001 Gew.‰ bis 1 Gew.% der Gesamtrestaurationsmasse eingesetzt.

Die Erfindung soll anhand der nachstehenden Beispiele näher erläutert werden. Zur Herstellung lichthärtender dentaler Composite mit verschiedenen farb-ästhetischen Beimengungen und anschließender Farbprüfung werden folgende Systeme (A, B) entsprechend dem Stand der Technik gemischt.

### Beispiel 1:

### A Restaurationsmaterial (Composite):

68 Gew.% silanisierte Glaskeramik
8 Gew.% Aerosil (mikrofeiner Füllstoff auf SiO₂-Basis)
18 Gew.% Bis-GMA (Bisphenol-A-Glycidyldimethacrylat)
5,3 Gew.% TEDMA (Triethylenglycoldimethacrylat)

### Stabilisatoren/Initiatoren:

0,3 Gew.% Campherchinon
0,3 Gew.% Dimethylaminobenzoesäure-ethylester
0,1 Gew.% BHT (Butylhydroxytoluol)
zusätzlich Farbmittel B entsprechend Tabelle 1:

**Tabelle 1**

| **Farbmittel B** | | |
|---|---|---|
| **Farbeffekt** | **Farbmittel, Zusammensetzung******* | **Nummer** |
| **goldfarben (fein, brillant)** | 0,047 Gew.‰ Iriodin® 302 (Korngröße 5-20 µm) | 1 |
| | 0,003 Gew.‰ Eisenoxidpigment (gelb) | |
| **zahnfleischfarben** | 0,05 Gew.‰ PV Echtrot B | 2 |
| | 0,087 Gew.‰ Titandioxidpigment (weiß) | |
| | 0,0125 Gew.‰ Eisenoxidpigment (gelb) | |
| | 0,0125 Gew.‰ Eisenoxidpigment (schwarz) | |
| **blauer Farbglanz** | 0,05 Gew.‰ SicopalBlau K7210 | 3 |
| **Glitzereffekt, Perlglanz** | 0,05 Gew.‰ Glimmer (silber, glänzend) | 4 |

| | | |
|---|---|---|
| * bezogen auf Gesamtrestaurationsmasse | | |

Die Restaurationssysteme A/B1, A/B2, A/B3 und A/B4 werden nach bekannten Verarbeitungsvorschriften in extrahierten Modellzähnen (Human- bzw. Rinderzähne) ausgehärtet. Die Modellrestaurationen A/B1 - A/B4 werden anschließend einer Farbüberprüfung und einem Farbvergleich unterzogen. Jede Farbe hat drei Dimensionen (Farbton, Sättigung und Helligkeit), die eine objektive Bestimmung und Beschreibung ermöglichen. Subjektiv ist Farbe aber kein Charakteristikum eines Objektes, sondern viel eher des Lichtes, welches vom Objekt her in unser Auge fällt. Das Sehen und Erkennen von Farben unterliegt daher den drei Faktoren Licht, Objekt und Betrachter.

Die Modellrestaurationen A/B1 (goldfarben) wurden neben einem mit einem Goldinlay restaurierten Zahn plaziert. Der objektive und subjektive Vergleich ergab eine nahezu identische Farbwirkung der Modellrestauration zum Goldinlay.

Mit dem Modellrestaurationmaterial A/B2 (zahnfleischfarben) wurde in eine Zahnhalskavität eines menschlichen Zahnes (Modellzahn) gefüllt. Im Vergleich zu natürlichem Zahnfleisch und zahnfleischfarbenen Kunstprothesen ergab sich eine identische Farbwirkung.

Die gefärbten Füllungsmaterialien A/B3 und A/B4 hoben sich charakteristisch von der normalen Zahnfarbe ab. A/B3 ist blau, A/B4 wirkt perlmutglänzend, ähnlich einer Metalliclackierung.

### Beispiel 2:

Die Füllungssysteme A/B1, A/B2, A/B3 und A/B4 wurden zur Überprüfung der physikalischen Eigenschaften üblichen Standarduntersuchungen unterzogen. Es wurden die Biegefestigkeit und die Löslichkeit entsprechend ISO 4049 im Vergleich zu zwei auf dem Markt erhältlichen dentalen Composite Materialien (C: Arabesk Top, VOCO GmbH; D: Tetric Ceram, Vivadent) sowie zu dem Restaurationsmaterial A/B nach Beispiel 1 ohne Zusatz von Farbmitteln untersucht. Die Untersuchungen ergaben folgende in Tabelle 2 dargestellten Ergebnisse:

**Tabelle 2**

| Biegefestigkeit und Löslichkeit dentaler Restaurationsmaterialien | | |
|---|---|---|
| **Restaurationsmaterial** | **Biegefestigkeit [MPa]** | **Löslichkeit [µg/mm**^{**3**}**]** |
| **AB1** | 142 | 0,15 |
| **AB2** | 141 | 0,15 |
| **AB3** | 142 | 0,16 |
| **AB4** | 145 | 0,13 |
| **A/B ohne Farbmittel** | 142 | 0,15 |
| **C** | 148 | 0,10 |
| **D** | 126 | 0,12 |

Es zeigt sich anhand der Beispiele, daß mit dem erfindungsgemäßen dentalen Restaurationsmaterial mit ästhetisch wirkenden Beimengungen von Farbmitteln die individuellen Wünsche und besonderen Gegebenheiten der zu behandelnden Patienten erfüllt werden können und es werden farbige, nicht zahnfarbene Restaurationen erhalten. Die Einarbeitung von Farbmitteln zur Erzeugung nicht zahnfarbener Farbwirkungen hat dabei keinerlei Einfluß auf die chemischen, physikalischen oder sonstigen Eigenschaften des Restaurationsmaterials.

## Patentansprüche

1. Farbiges, nicht zahnfarbenes, dentales Restaurationsmaterial in das neben weiteren üblichen Bestandteilen anorganische und/oder organische Farbmittel zur Erzielung einer nicht zahnfarbenen Farbwirkung eingearbeitet sind.

2. Dentales Restaurationsmaterial nach Anspruch 1, dadurch gekennzeichnet, daß durch die Farbmittel eine farbige, nicht zahnfarbene, Anpassung des Restaurationsmaterials an die Zahnumgebung erreicht wird.

3. Dentales Restaurationsmaterial nach Anspruch 2, dadurch gekennzeichnet, daß Farbmittel eingearbeitet sind, so daß eine farbige Anpassung an das Zahnfleisch erreicht wird.

4. Dentales Restaurationsmaterial nach Anspruch 2, dadurch gekennzeichnet, daß Farbmittel eingearbeitet sind, so daß eine farbige Anpassung an goldfarbene Restaurationen erreicht wird.

5. Dentales Restaurationsmaterial nach Anspruch 1, dadurch gekennzeichnet, daß Farbmittel eingearbeitet sind, so daß ein gelbes, grünes, blaues, rotes oder durch Mischung mehrerer Farbmittel bunt gefärbtes Restaurationsmaterial vorliegt.

6. Dentales Restaurationsmaterial nach Anspruch 1, dadurch gekennzeichnet, daß Farbmittel eingearbeitet sind, so daß das Restaurationsmaterial einen Glimmer- oder glimmerartigen Anteil aufweist.

7. Dentales Restaurationsmaterial nach einem der Ansprüche 1 bis 6, dadurch gekennzeichnet, daß das Restaurationsmaterial mit den Farbmitteln durchgefärbt ist.

8. Dentales Restaurationsmaterial nach einem der Ansprüche 1 bis 7, dadurch gekennzeichnet, daß die eingesetzten Farbmittel in Mengen von 0,001 Gew.‰ bis 1 Gew.% der Gesamtrestaurationsmasse eingesetzt werden.

9. Dentales Restaurationsmaterial nach einem der Ansprüche 1 bis 8, dadurch gekennzeichnet, daß es sich um ein plastisch verformbares Restaurationsmaterial handelt.

10. Dentales Restaurationsmaterial nach Anspruch 9, dadurch gekennzeichnet, daß es sich um ein Composite-Material handelt.

11. Dentales Restaurationsmaterial nach Anspruch 9, dadurch gekennzeichnet, daß es sich um ein Glasionomerzement handelt.

12. Dentales Restaurationsmaterial nach einem der Ansprüche 1 bis 11, dadurch gekennzeichnet, daß die chemischen und physikalischen Eigenschaften des Restaurationsmaterials, wie insbesondere die Festigkeits-, Löslichkeits- und Verarbeitungseigenschaften, nicht durch die Farbmittel beeinflußt werden.

13. Verfahren zur Herstellung farbiger, nicht zahnfarbener, dentaler Restaurationsmaterialien nach einem der Ansprüche 1 bis 12, dadurch gekennzeichnet, daß in das Restaurationsmaterial neben weiteren üblichen Bestandteilen anorganische und/oder organische Farbmittel zur Erzielung einer nicht zahnfarbenen Farbwirkung eingearbeitet werden.

14. Verwendung farbiger, nicht zahnfarbener, dentaler Restaurationsmaterialien nach einem der Ansprüche 1 bis 12, zum Ersetzen oder Restaurieren von Zähnen, dadurch gekennzeichnet, daß in das Restaurationsmaterial neben weiteren üblichen Bestandteilen anorganische und/oder organische Farbmittel zur Erzielung einer nicht zahnfarbenen Farbwirkung eingearbeitet sind.
